# FASCICULE DE BREVET EUROPEEN

(11) **EP 2 303 834 B1**
(45) Date de publication et mention de la délivrance du brevet: **19.09.2018**
(21) Numéro de dépôt: 09765752.2
(22) Date de dépôt: 05.06.2009
(51) Int. Cl.: C07C 253/10, B01J 31/24, C07C 255/04

(54) **PROCEDE DE FABRICATION DE COMPOSES NITRILES A PARTIR DE COMPOSES A INSATURATION ETHYLENIQUE**
VERFAHREN ZUR HERSTELLUNG VON NITRILVERBINDUNGEN AUS ETHYLENISCH UNGESÄTTIGTEN VERBINDUNGEN
PROCESS FOR PRODUCING NITRILE COMPOUNDS FROM ETHYLENICALLY UNSATURATED COMPOUNDS

(30) Priorité: 17.06.2008 FR 0803373
(43) Date de publication de la demande: 06.04.2011
(73) Titulaire: INVISTA Textiles (U.K.) Limited, Manchester M2 3DE (GB)
(72) Inventeur: MASTROIANNI, Sergio, F-69009 Lyon (FR)
(74) Mandataire: Carpmaels & Ransford LLP
(86) Numéro de dépôt international: PCT/EP2009/056917
(87) Numéro de publication internationale: WO 2009/153172

(56) Documents cités:
- WO-A-02/053527
- FR-A- 2 830 530
- US-A- 6 153 758
- US-A1- 2004 116 713

## Description

La présente invention concerne un procédé d'hydrocyanation en dinitriles de composés nitriles à insaturation éthylénique choisis parmi le pentène-3-nitrile, le pentène-4-nitrile et leurs mélanges.

Le brevet français n° 1 599 764 décrit un procédé de préparation de nitriles par addition d'acide cyanhydrique sur des composés organiques ayant au moins une double liaison éthylénique, en présence d'un catalyseur comprenant du nickel et un ligand organophosphorés, une triarylphosphite. Cette réaction peut être conduite en présence ou non d'un solvant.

Quand un solvant est utilisé, il s'agit de préférence d'un hydrocarbure, tel que le benzène ou les xylènes ou d'un nitrile tel que l'acétonitrile.

Le catalyseur mis en oeuvre est un complexe organique de nickel, contenant des ligands tels que les phosphines, les arsines, les stibines, les phosphites, les arsénites ou les antimonites.

La présence d'un promoteur pour activer le catalyseur, tel qu'un composé du bore ou un sel métallique, généralement un acide de Lewis, est également préconisée dans ledit brevet.

De nombreux autres systèmes catalytiques ont été proposés comprenant généralement des composés organophosphorés appartenant à la famille des phosphites, phosphonites, phosphinites et phosphines. Ces composés organophosphorés peuvent comprendre un atome de phosphore par molécule et sont qualifiés de ligands monodentates. Ils peuvent comprendre plusieurs atomes de phosphore par molécules, ils sont alors appelés ligands pluridentates, plus particulièrement de nombreux ligands contenant deux atomes de phosphore par molécules (ligand bidentate) ont été décrits dans de nombreux brevets. Le document WO02/053527 décrit un procédé d'hydrocyanation avec un ligand furylphosphine.

Toutefois, la recherche de nouveaux systèmes catalytiques plus performants tant en activité catalytique qu'en stabilité est toujours entreprise.

Un des buts de la présente invention est de proposer une nouvelle famille de ligands qui permet d'obtenir avec les métaux de transition des systèmes catalytiques présentant une bonne activité catalytique dans la réaction d'hydrocyanation.

A cet effet, la présente invention propose un procédé d'hydrocyanation en dinitriles de composés nitriles à insaturation éthylénique choisis parmi le pentène-3-nitrile, le pentène-4-nitrile et leurs mélanges par réaction en milieu liquide avec le cyanure d'hydrogène en présence d'un système catalytique comprenant un cocatalyseur consistant en au moins un acide de Lewis et un catalyseur comprenant le nickel et un ligand organophosphorés caractérisé en ce que le ligand organophosphoré comprend au moins un compose choisi parmi la (2-thiényl)diphénylphosphine, la di(2-thiényl)phénylphosphine, la tri(2-thiényl)phosphine, la (2-pyrryl)diphénylphosphine, la di(2-pyrryl)phénylphosphine, la tri(2-pyrryl)phosphine, la (2-pyridyl)diphénylphosphine, et la di(2-pyridyl)phénylphosphine, la tri(2-pyridyl)phosphine.

Avantageusement, le ligand organophosphoré est un compose correspondant à ceux précédemment décrit) ou un mélange de composes organophosphines monodentates dont au moins un compose répondant à ceux précédemment décrit.

Selon l'invention, la composition du système catalytique peut être représentée par la formule générale (II) (cette formule ne correspond pas à la structure des composes et complexes présents dans le système catalytique):

M [L_{f}]ₜ (II)

Dans laquelle:
M est le nickel,
L_{f} représente au moins un ligand organophosphoré dont au moins un correspond à un composé choisi parmi la (2-thiényl)diphénylphosphine, la di(2-thiényl)phénylphosphine, la tri(2-thiényl)phosphine, la (2-pyrryl)diphénylphosphine, la di(2-pyrryl)phénylphosphine, la tri(2-pyrryl)phosphine, la (2-pyridyl)diphénylphosphine, et la di(2-pyridyl)phénylphosphine, la tri(2-pyridyl)phosphine,
t représente un nombre compris entre 1 et 10 (bornes incluses).
Le nickel est l'élément préfère pour l'hydrocyanation des oléfines et des nitriles insaturés.

Pour la préparation des thiénylphosphines et pyrrylphoshines on peut se référer par exemple à l'article de V.K. Issleib et A. Brack publie dans Zeitschrift fur anorganische und allgemeine Chemie, 1957, 292, pages 245 a 253. Pour la synthèse des pyridylphosphines selon la formule générale (I) on peut se référer par exemple au brevet EP0499328.

La préparation des systèmes catalytiques comprenant des ligands organophosphorés éventuellement en mélange avec d'autres organophosphines monodentates peut être effectuée en mettant en contact une solution d'un compose du nickel, avec une solution du compose organophosphoré de l'invention.

Le compose du nickel peut être dissous dans un solvant. Le nickel peut se trouver dans le compose mis en oeuvre, soit au degré d'oxydation qu'il aura dans le complexe organométallique, soit à un degré d'oxydation supérieur.

A titre d'exemple, on peut indiquer que dans les complexes organométalliques de l'invention, le nickel est au degré d'oxydation (0).

Si lors de la préparation du complexe organométallique, le nickel est mis en oeuvre à un degré d'oxydation plus élevé, il pourra être réduit in situ.

Parmi les composes de nickel utilisables pour la préparation des complexes organométalliques on peut citer, à titre d'exemples non limitatifs, les composes suivants:
- les composes dans lesquels le nickel est au degré d'oxydation zéro comme le tétracyanonickelate de potassium K₄ [Ni(CN)₄], le bis (acrylonitrile) nickel zéro, le bis (cyclooctadiène-1,5) nickel (appelé également Ni(cod)₂) et les dérivés contenant des ligands comme le tétrakis (triphényl phosphine) nickel zéro.
- les composés du nickel comme les carboxylates (notamment l'acétate), carbonate, bicarbonate, borate, bromure, chlorure, citrate, thiocyanate, cyanure, formiate, hydroxyde, hydrophosphite, phosphite, phosphate et dérivés, iodure, nitrate, sulfate, sulfite, aryl- et alkyl-sulfonates.

Quand le composé du nickel utilisé correspond à un état d'oxydation du nickel supérieur à 0, on ajoute au milieu réactionnel un réducteur du nickel réagissant préférentiellement avec celui-ci dans les conditions de la réaction. Ce réducteur peut être organique ou minéral. On peut citer comme exemples non limitatifs les borohydrures comme le BH₄Na, le BH₄K, la poudre de Zn, le magnésium ou l'hydrogène.

Quand le composé du nickel utilisé correspond à l'état d'oxydation 0 du nickel, on peut également ajouter un réducteur du type de ceux précités, mais cet ajout n'est pas impératif.

Les pentène-nitriles peuvent contenir, en plus du pentène-3-nitrile et du pentène-4-nitrile, des quantités, généralement minoritaires, d'autres composés, comme le méthyl-2-butène-3-nitrile, le méthyl-2-butène-2-nitrile, le pentène-2-nitrile, le valéronitrile, l'adiponitrile, le méthyl-2-glutaronitrile, l'éthyl-2-succinonitrile ou le butadiène, provenant par exemple de la réaction antérieure d'hydrocyanation du butadiène en nitriles insaturés.

En effet, lors de l'hydrocyanation du butadiène, il se forme avec les pentène-nitriles linéaires des quantités non négligeables de méthyl-2-butène-3-nitrile et de méthyl-2-butène 2-nitrile.

Le système catalytique utilisé pour l'hydrocyanation selon le procédé de l'invention peut être prépare avant son introduction dans la zone de réaction, par exemple par addition au ligand organophosphoré seule ou dissoute dans un solvant, la quantité appropriée de composé du métal de transition choisi et éventuellement du réducteur. Il est également possible de préparer le système catalytique "in situ" par simple addition de la phosphine et du compose du métal de transition dans le milieu réactionnel d'hydrocyanation avant ou après l'addition du composé à hydrocyaner.

La quantité de compose du nickel est choisie pour obtenir une concentration en mole de métal de transition par mole de composés nitriles à hydrocyaner comprise entre 10⁻⁴ et 1, et de préférence entre 0,005 et 0,5 mole de nickel.

La quantité de composés organophosphines utilisée pour former le catalyseur est choisie de telle sorte que le nombre de moles de ce composé rapporte a 1 mole de métal de transition soit de 0,5 à 100 et de préférence de 2 à 50.

Bien que la réaction soit conduite généralement sans solvant, il peut être avantageux de rajouter un solvant organique inerte. Le solvant peut être un solvant du catalyseur qui est miscible a la phase comprenant le composé à hydrocyaner à la température d'hydrocyanation. A titre
d'exemples de tels solvants, on peut citer les hydrocarbures aromatiques, aliphatiques ou cycloaliphatiques.

La réaction d'hydrocyanation est généralement réalisée à une température de 10° C à 200° C et de préférence de 30° C à 120° C. Elle peut être réalisée en milieu monophasique.

Le procède de l'invention peut être mis en oeuvre de manière continue ou discontinue.

Le cyanure d'hydrogène mis en oeuvre peut être prépare à partir des cyanures métalliques, notamment le cyanure de sodium, ou des cyanhydrines, comme la cyanhydrine de l'acétone ou par tout autre procédé de synthèse connu tel que le procédé Andrussov consistant à faire réagir le méthane avec l'ammoniac et de l'air.

Le cyanure d'hydrogène exempt d'eau est introduit dans le réacteur sous forme gazeuse ou sous forme liquide. Il peut également être préalablement dissous dans un solvant organique.

Dans le cadre d'une mise en oeuvre discontinue, on peut en pratique charger dans un réacteur, préalablement purgé à l'aide d'un gaz inerte (tel qu'azote, argon), soit une solution contenant la totalité ou une partie des divers constituants tels que le ligand organophosphoré, le nickel, les éventuels réducteur et solvant, soit séparément lesdits constituants. Généralement le réacteur est alors porté à la température choisie, puis le composé à hydrocyaner est introduit. Le cyanure d'hydrogène est alors lui-même introduit, de préférence de manière continue et régulière.

Quand la réaction (dont on peut suivre l'évolution par dosage de prélèvements) est terminée, le mélange réactionnel est soutiré après refroidissement et les produits de la réaction sont isolés et séparés, par exemple, par distillation.

Avantageusement, la synthèse de dinitriles tels que l'adiponitrile à partir de dioléfines (butadiène) est obtenue en deux étapes successives. La première étape consiste à hydrocyaner une double liaison de la dioléfine pour obtenir un mononitrile insaturé. La seconde étape consiste à hydrocyaner l'insaturation du mononitrile pour obtenir le ou les dinitriles correspondants et peut répondre au procédé de l'invention. Ces deux étapes sont généralement mises en oeuvre avec un système catalytique comprenant un complexe organométallique de même nature. Toutefois, les rapports composé organophosphoré/ élément métallique et concentration du catalyseur peuvent être différents. En outre, il est associé au système catalytique un cocatalyseur dans la seconde étape. Ce cocatalyseur est un acide de Lewis.

L'acide de Lewis utilisé comme cocatalyseur permet notamment, dans le cas de l'hydrocyanation des nitriles aliphatiques à insaturation éthylénique, d'améliorer la linéarité des dinitriles obtenus, c'est-à-dire le pourcentage de dinitrile linéaire par rapport à la totalité des dinitriles formes, et/ou d'augmenter l'activité et la durée de vie du catalyseur.

Par acide de Lewis, on entend dans le présent texte, selon la définition usuelle, des composés accepteurs de doublets électroniques.

On peut mettre en oeuvre notamment les acides de Lewis cités dans l'ouvrage édité par G.A. OLAH "Friedel-Crafts and related Reactions", tome I, pages 191 à 197 (1963).

Les acides de Lewis qui peuvent être mis en oeuvre comme cocatalyseurs dans le présent procédé sont choisis parmi les composés des éléments des groupes Ib, IIb, IIIa, IIIb, IVa, IVb, Va, Vb, VIb, VIIb et VIII de la Classification périodique des éléments. Ces composes sont le plus souvent des sels, notamment des halogénures, comme chlorures ou bromures, sulfates, sulfonates, halogenosulfonates, perhalogénoalkyl sulfonates, notamment fluoroalkylsulfonates ou perfluoroalkylsulfonates, carboxylates et phosphates.

A titre d'exemples non limitatifs de tels acides de Lewis, on peut citer le chlorure de zinc, le bromure de zinc, l'iodure de zinc, le chlorure de manganèse, le bromure de manganèse, le chlorure de cadmium, le bromure de cadmium, le chlorure stanneux, le bromure stanneux, le sulfate stanneux, le tartrate stanneux, le trifluorométhylsulfonate d'indium, les chlorures ou bromures des éléments des terres rares comme le lanthane, le cérium, le praséodyme, le néodyme, le samarium, l'europium, le gadolinium, le terbium, le dysprosium, l'hafnium, l'erbium, le thallium, l'ytterbium et le lutétium, le chlorure de cobalt, le chlorure ferreux, le chlorure d'yttrium.

On peut également utiliser comme acide de Lewis des composes organométalliques comme le triphénylborane, l'isopropylate de titane ou les composés décrits dans le brevet français n°2926816.

On peut bien entendu mettre en oeuvre des mélanges de plusieurs acides de Lewis.

Parmi les acides de Lewis, on préfère tout particulièrement le chlorure de zinc, le bromure de zinc, le chlorure stanneux, le bromure stanneux le triphénylborane et les mélanges chlorure de zinc/chlorure stanneux.

Le cocatalyseur acide de Lewis mis en oeuvre représente généralement de 0,01 à 50 moles par mole de composé du nickel, et de préférence de 1 à 10 mole par mole.

Les mononitriles insaturés mis en oeuvre dans cette deuxième étape sont des pentène-nitriles linéaires choisis parmi le pentène-3-nitrile, le pentène-4-nitrile et leurs mélanges.

Ces pentène-nitriles peuvent contenir des quantités, généralement minoritaires, d'autres composes, comme le méthyl-2-butène-3-nitrile, le méthyl-2-butène-2-nitrile, le pentène-2-nitrile.

La solution catalytique utilisée pour l'hydrocyanation en présence d'acide de Lewis peut être préparée avant son introduction dans la zone de réaction, par exemple par addition a la phosphine de formule (I), de la quantités appropriée de composé du métal de transition choisi, de l'acide de Lewis et éventuellement du réducteur. Il est également possible de préparer la solution catalytique "in situ" par simple addition de ces divers constituants dans le milieu réactionnel.

Il est également possible dans les conditions du procédé d'hydrocyanation, et notamment en opérant en présence du catalyseur décrit précédemment comportant au moins un ligand organophosphoré et au moins un composé d'un métal de transition, de réaliser, en absence de cyanure d'hydrogène, l'isomérisation du méthyl-2-butène-3-nitril en pentènenitriles, et plus généralement des nitriles insaturés ramifies en nitriles insaturés linéaires.

Le méthyl-2-butène-3-nitril soumis à l'isomérisation peut être mis en oeuvre seul ou en mélange avec d'autres composés. Ainsi on peut engager du méthyl-2-butène-3-nitril en mélange avec du méthyl-2-butène-2-nitril, du pentène-4-nitrile, du pentène-3-nitrile, du pentène-2-nitrile, du butadiène.

Il est particulièrement intéressant de traiter le mélange réactionnel provenant de l'hydrocyanation du butadiène par HCN en présence d'au moins un ligand organophosphoré et d'au moins un composé d'un métal de transition, plus préférentiellement d'un composé du nickel au degré d'oxydation 0, tel que défini précédemment. Dans le cadre de cette variante, le system catalytique étant déjà présent pour la réaction d'hydrocyanation du butadiène, il suffit d'arrêter toute introduction de cyanure d'hydrogène, pour laisser se produire la réaction d'isomérisation. On peut, le cas échéant, dans cette variante faire un léger balayage du réacteur à l'aide d'un gaz inerte comme l'azote ou l'argon par exemple, afin de chasser l'acide cyanhydrique qui pourrait être encore présent.

La réaction d'isomérisation est généralement réalisée à une température comprise entre 10°C et 200° C et de préférence entre 60° C et 140° C.

Dans le cas d'une isomérisation suivant immédiatement la réaction d'hydrocyanation du butadiène, ii sera avantageux d'opérer à la température à laquelle l'hydrocyanation a été conduite ou légèrement supérieure.

Comme pour le procédé d'hydrocyanation de composés a insaturation éthylénique, le system catalytique utilisé pour l'isomérisation peut être prépare avant son introduction dans la zone de réaction, par exemple par mélange du ligand organophosphoré, de la quantité appropriée de composé du métal de transition choisi et éventuellement du réducteur. Il est également possible de préparer le system catalytique "in situ" par simple addition de ces divers constituants dans le milieu réactionnel. La quantité de composé du métal de transition et plus particulièrement du nickel utilisée, ainsi que la quantité de ligand organophosphoré sont les mêmes que pour la réaction d'hydrocyanation.

Bien que la réaction d'isomérisation soit conduite généralement sans solvant, il peut être avantageux de rajouter un solvant organique inerte qui pourra être utilisé comme solvant d'extraction, ultérieurement. C'est notamment le cas lorsqu'un tel solvant a été mis en oeuvre dans la réaction d'hydrocyanation du butadiène ayant servi à préparer le milieu soumis à la réaction d'isomérisation. De tels solvants peuvent être choisis parmi ceux qui ont été cités précédemment pour l'hydrocyanation.

Toutefois, la préparation de composés dinitriles par hydrocyanation d'une oléfine comme le butadiène peut être réalisée en utilisant un système catalytique conforme à l'invention pour les étapes de formation des nitriles insaturés et l'étape d'isomérisation ci-dessus.

De même, la réaction d'hydrocyanation de l'oléfine en nitriles insaturés et l'isomérisation de ceux-ci peuvent être réalisée avec un système catalytique différent de celui de l'invention, l'étape d'hydrocyanation des nitriles insaturés en dinitriles étant mis en oeuvre avec un système catalytique conforme à l'invention.

D'autres détails, avantages de l'invention seront illustrés par les exemples donnés ci-dessous uniquement à titre indicatif, sans caractère limitatif.

### Abréviations utilisées

- Cod: cyclooctadiène
- Ni(Cod)₂ : bis(1,5-cyclooctadiène)nickel
- 3PN: 3-pentènenitrile
- AdN: Adiponitrile
- ESN: éthylsuccinonitrile
- MGN: méthylglutaronitrile
- DN : composés dinitriles (AdN, MGN ou ESN)
- TIBAO: tetraisobutyldialuminoxane
- Mes: groupement mésityle (2,4,6 triméthylphényle)
- Ph: groupement phényle
- RR(DN): rendement réel de dinitriles correspondant au rapport du nombre de moles formées de dinitriles sur le nombre de moles de 3PN engagé
- Linéarité (L): rapport du nombre de moles d'AdN formées sur le nombre de moles de dinitriles formées (somme des moles d'AdN, ESN et MGN)
Les composés suivants: 3PN, Ni(Cod)₂, ZnCl2, TiBAO, anhydride diphénylborinique (Ph₂BOPh₂), tris(2-thiényl)phosphine, tris(2-furyl)phosphine, (2-pyridyl)diphénylphosphine, sont des produits connus et disponibles commercialement.

### Exemples 0 à 7 : Hydrocyanation du 3-PN en AdN

Les essais sont réalisés selon le mode opératoire suivant :
Sous atmosphère d'argon, dans un tube de verre type Shott de 60 ml équipé d'un bouchon-septum, sont chargés successivement :
le ligand A de formule I (2,5 mmol, soit 5 équivalents en P)
3PN anhydre : 1,21 g (15 mmol, soit 30 équivalents)
Ni(cod)₂:138 mg (0,5 mmol, 1 équivalent)
acide de Lewis quantité et nature indiquées dans le tableau I ci-dessous.

Les ligands A utilisés dans les exemples sont :
- Tris(2-thiényl)phosphine : commercialisé par la société Aldrich 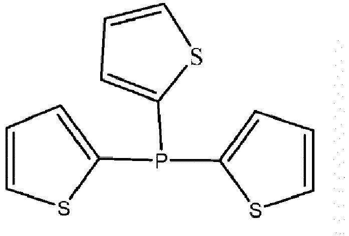 (2-pyridyl)diphénylphosphine: commercialisé par la société Aldrich

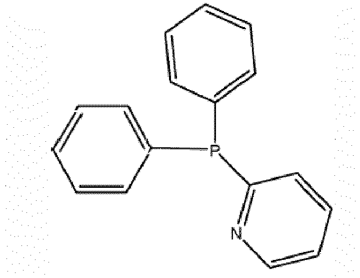

Le mélange est porté sous agitation à 70°C. La cyanhydrine de l'acétone est injectée dans le milieu réactionnel par un pousse-seringue à un débit de 0,45 ml par heure. Après 3 heures, l'injection est arrêtée. Le mélange est refroidi à température ambiante, dilué a l'acétone et analysé par chromatographie en phase gazeuse.
Les résultats sont regroupés dans le tableau suivant

**Tableau 1: exemples 0 à 7**

| exemple | Ligand A | Acide de Lewis | Acide de Lewis/Ni | Linéarité | RR (DN) |
|---|---|---|---|---|---|
| 0 (comparatif) | Tris(2-furyl)phosphine | TiBAO | 0,5 | 52,7 | 30,4 |
| 1 | Tris(2-thiényl)phosphine | ZnCl₂ | 1 | 62,5 | 75,8 |
| 2 | Tris(2-thiényl)phosphine | TiBAO | 0,5 | 64,1 | 70,6 |
| 3 | Tris(2-thiényl)phosphine | Ph₂BOBPh₂ | 0,5 | 84,7 | 32,3 |
| 4 | Tris(2-thiényl)phosphine | Mes₂BOZnEt* | 0,5 | 65 | 9,2 |
| 5 | (2-pyridyl)diphénylphosphine | ZnCl₂ | 1 | 63,6 | 9,5 |
| 6 | (2-pyridyl)diphénylphosphine | TiBAO | 0,5 | 62,8 | 16,5 |
| 7 | (2-pyridyl)diphénylphosphine | Ph₂BOBPh₂ | 0,5 | 74,4 | 8,1 |

| | | | | | |
|---|---|---|---|---|---|
| * La synthèse de cet acide de Lewis est décrite dans le brevet français numéro 2926816. | | | | | |

### Exemple 8 hydrocyanation du 3-PN en AdN

Sous atmosphère d'argon, dans un tube de verre type Shott de 60 ml équipé d'un bouchon-septum, sont chargés successivement :
- tris(2-thiényl)phosphine (1,25 mmol, soit 2,5 équivalents en P)
- tris(2-furyl)phosphine (1,25 mmol, soit 2,5 équivalents en P)
- 3PN anhydre : 1,21 g (15 mmol, soit 30 équivalents)
- Ni(cod)₂:138 mg (0,5 mmol, 1 équivalent)
- anhydride diphénylborinique 91 mg (0,25 mmol, soit 0,5 équivalent) comme acide de Lewis
Le mélange est porté sous agitation à 70°C. La cyanhydrine de l'acétone est injectée dans le milieu réactionnel par un pousse-seringue à un débit de 0,45 ml par heure. Après 3 heures, l'injection est arrêtée. Le mélange est refroidi à température ambiante, dilué à l'acétone et analysé par chromatographie en phase gazeuse.
La linéarité mesurée est de 87,6% pour un rendement en dinitriles RR(DN) de 22,2%

## Revendications

1. Procédé d'hydrocyanation en dinitriles de composés nitriles à insaturation éthylénique choisis parmi le pentène-3-nitrile, le pentène-4-nitrile et leurs mélanges par réaction en milieu liquide avec le cyanure d'hydrogène en présence d'unsystème catalytique comprenant un cocatalyseur consistant en au moins un acide de Lewis et uncatalyseur comprenant le nickel et un ligand organophosphoré **caractérisé en ce que** le ligand organophosphoré comprend au moins un composéchoisi parmi la (2-thiényl)diphénylphosphine, la di(2-thiényl)phénylphosphine, la tri(2-thiényl)phosphine, la (2-pyrryl)diphénylphosphine, la di(2-pyrryl)phénylphosphine, la tri(2-pyrryl)phosphine, la (2-pyridyl)diphénylphosphine, et la di(2-pyridyl)phénylphosphine, la tri(2-pyridyl)phosphine.

2. Procédé selon la revendication 1, **caractérisé en ce que** le ligand organophosphoré répond à une des formules suivantes :

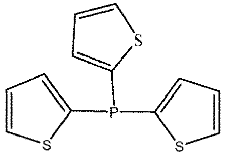

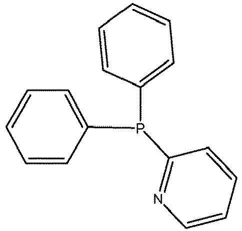

3. Procédé selon l'une des revendications précédentes **caractérisé en ce que** la composition du catalyseur est exprimée par la formule générale (II):
M [L_{f}]ₜ (II)
Dans laquelle:
M est le nickel,
L_{f} représente le ou les ligands organophosphorés dont au moins un correspond à un composé choisi parmi la (2-thiényl)diphénylphosphine, la di(2-thiényl)phénylphosphine, la tri(2-thiényl)phosphine, la (2-pyrryl)diphénylphosphine, la di(2-pyrryl)phénylphosphine, la tri(2-pyrryl)phosphine, la (2-pyridyl)diphénylphosphine, et la di(2-pyridyl)phénylphosphine, la tri(2-pyridyl)phosphine,
t représente un nombre compris entre 1 et 10 inclus.

4. Procédé selon l'une des revendications précédentes, **caractérisé en ce que** la quantité de composé du nickel utilisée est choisie de telle sorte qu'il y ait par mole de composé organique à hydrocyaner entre 10⁻⁴ et 1 mole de nickel mis en oeuvre et **en ce que** la quantité de composés organosphosphorés utilisée est choisie de telle sorte que le nombre de moles de ces composés rapporté à 1 mole de nickel soit de 0,5 à 100.

5. Procédé selon l'une des revendications précédentes, **caractérisé en ce que** l'acide de Lewis mis en oeuvre comme cocatalyseur est choisi parmi les composés des éléments des groupes Ib, IIb, IIIa, IIIb, IVa, IVb, Va, Vb, VIb, VIIb et VIII de la Classification périodique des éléments.

6. Procédé selon l'une des revendications précédentes, **caractérisé en ce que** l'acide de Lewis est choisi parmi le chlorure de zinc, le bromure de zinc, l'iodure de zinc, le chlorure de manganèse, le bromure de manganèse, le chlorure de cadmium, le bromure de cadmium, le chlorure stanneux, le bromure stanneux, le sulfate stanneux, le tartrate stanneux, le trifluorométhylsulfonate d'indium, les chlorures ou bromures des éléments des terres rares comme le lanthane, le cérium, le praséodyme, le néodyme, le samarium, l'europium, le gadolinium, le terbium, le dysprosium, l'hafnium, l'erbium, le thallium, l'ytterbium et le lutétium, le chlorure de cobalt, le chlorure ferreux, le chlorure d'yttrium et leurs mélanges, les composés organométalliques.

## Patentansprüche

1. Verfahren zur Hydrocyanierung von ethylenisch ungesättigten Nitrilverbindungen, die aus 3-Pentennitril, 4-Pentennitril und Mischungen davon ausgewählt sind, zu Dinitrilverbindungen durch Umsetzung mit Cyanwasserstoff in flüssigem Medium in Gegenwart eines katalytischen Systems, das einen Cokatalysator, der zumindest aus einer Lewis-Säure besteht, und einen Katalysator, der Nickel und einen Organophosphorliganden umfasst, umfasst, **dadurch gekennzeichnet, dass** der Organophosphorligand mindestens eine aus (2-Thienyl)-diphenylphosphin, Di(2-thienyl)phenylphosphin, Tri(2-thienyl)phosphin, (2-Pyrryl)diphenyl-phosphin, Di(2-pyrryl)phenylphosphin, Tri(2-pyrryl)phosphin, (2-Pyridyl)diphenylphosphin, Di-(2-pyridyl)phenylphosphin und Tri(2-pyridyl)-phosphin ausgewählte Verbindung umfasst.

2. Verfahren nach Anspruch 1, **dadurch gekennzeichnet, dass** der Organophosphorligand einer der folgenden Formeln entspricht:

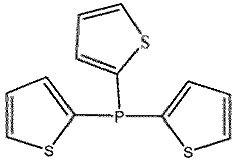

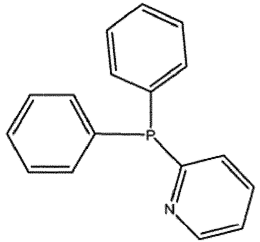

3. Verfahren nach einem der vorhergehenden Ansprüche, **dadurch gekennzeichnet, dass** die Zusammensetzung des Katalysators durch die allgemeine Formel (II) ausgedrückt wird:
M[L_{f}]ₜ (II)
worin:
M für Nickel steht,
L_{f} für den Organophosphorliganden bzw. die Organophosphorliganden steht, wovon mindestens einer einer aus (2-Thienyl)diphenylphosphin, Di(2-thienyl)phenylphosphin, Tri(2-thienyl)phosphin, (2-Pyrryl)diphenylphosphin, Di(2-pyrryl)phenylphosphin, Tri(2-pyrryl)phosphin, (2-Pyridyl)diphenylphosphin, Di(2-pyridyl)phenyl-phosphin und Tri(2-pyridyl)phosphin ausgewählten Verbindung entspricht,
t für eine Zahl zwischen 1 und 10 inklusive steht.

4. Verfahren nach einem der vorhergehenden Ansprüche, **dadurch gekennzeichnet, dass** man die verwendete Menge der Nickelverbindung so wählt, dass pro Mol zu hydrocyanierende organische Verbindung zwischen 10⁻⁴ und 1 mol Nickel verwendet werden, und die verwendete Menge von Organophosphorverbindungen so wählt, dass die Zahl der Mole dieser Verbindungen, bezogen auf 1 mol Nickel, 0,5 bis 100 beträgt.

5. Verfahren nach einem der vorhergehenden Ansprüche, **dadurch gekennzeichnet, dass** man die als Cokatalysator verwendete Lewis-Säure aus Verbindungen von Elementen der Gruppen Ib, IIb, IIIa, IIIb, IVa, IVb, Va, Vb, VIb, VIIb und VIII des Periodensystems der Elemente auswählt.

6. Verfahren nach einem der vorhergehenden Ansprüche, **dadurch gekennzeichnet, dass** man die Lewis-Säure aus Zinkchlorid, Zinkbromid, Zinkiodid, Manganchlorid, Manganbromid, Cadmiumchlorid, Cadmiumbromid, Zinn(II)-chlorid, Zinn(II)-bromid, Zinn(II)-sulfat, Zinn(II)-tartrat, Indiumtrifluormethylsulfonat, den Chloriden oder Bromiden der Seltenerdmetalle wie Lanthan, Cer, Praseodym, Neodym, Samarium, Europium, Gadolinium, Terbium, Dysprosium, Hafnium, Erbium, Thallium, Ytterbium und Lutetium, Cobaltchlorid, Eisen(II)-chlorid, Yttriumchlorid und Mischungen davon und Organometallverbindungen auswählt.

## Claims

1. Process for the hydrocyanation, to give dinitriles, of ethylenically unsaturated nitrile compounds chosen from 3-pentenenitrile, 4-pentenenitrile, and mixtures thereof, by reaction, in a liquid medium, with hydrogen cyanide in the presence of a catalytic system comprising a cocatalyst consisting of at least one Lewis acid and a catalyst comprising nickel and an organophosphorus ligand, **characterized in that** the organophosphorus ligand comprises at least one compound chosen from (2-thienyl)diphenylphosphine, di(2-thienyl)phenylphosphine, tri(2-thienyl)phosphine, (2-pyrryl)diphenylphosphine, di(2-pyrryl)phenylphosphine, tri(2-pyrryl)-phosphine, (2-pyridyl)diphenylphosphine, di(2-pyridyl)-phenylphosphine and tri(2-pyridyl)phosphine.

2. Process according to Claim 1, **characterized in that** the organophosphorus ligand corresponds to one of the following formulae:

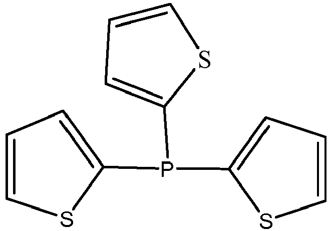

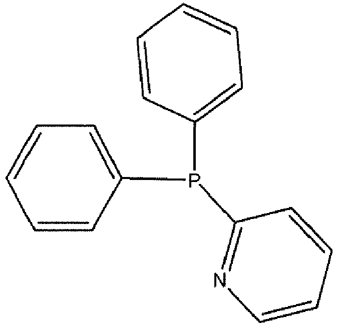

3. Process according to either of the preceding claims, **characterized in that** the composition of the catalyst is expressed by the general formula (II):
M[L_{f}]ₜ (II)
in which:
M is nickel,
L_{f} represents the organophosphorus ligand(s), at least one of which corresponds to a compound chosen from 2-thienyl)diphenylphosphine, di(2-thienyl)phenylphosphine, tri(2-thienyl)phosphine, (2-pyrryl)diphenylphosphine, di(2-pyrryl)phenylphosphine, tri(2-pyrryl)-phosphine, (2-pyridyl)diphenylphosphine, di(2-pyridyl)-phenylphosphine and tri(2-pyridyl)phosphine,
t represents a number between 1 and 10 inclusive.

4. Process according to one of the preceding claims, **characterized in that** the amount of nickel used is chosen such that between 10⁻⁴ and 1 mol of nickel are used per mole of organic compound to be hydrocyanated, and **in that** the amount of organophosphorus compounds used is chosen such that the number of moles of these compounds relative to 1 mol of nickel is from 0.5 to 100.

5. Process according to one of the preceding claims, **characterized in that** the Lewis acid used as cocatalyst is chosen from the compounds of elements from groups Ib, IIb, IIIA, IIIb, IVa, IVb, Va, Vb, VIb, VIIb and VIII of the Periodic Table of Elements.

6. Process according to one of the preceding claims, **characterized in that** the Lewis acid is chosen from zinc chloride, zinc bromide, zinc iodide, manganese chloride, manganese bromide, cadmium chloride, cadmium bromide, stannous chloride, stannous bromide, stannous sulfate, stannous tartrate, indium trifluoromethylsulfonate, chlorides or bromides of rare earth elements such as lanthanum, cerium, praseodymium, neodymium, samarium, europium, gadolinium, terbium, dysprosium, hafnium, erbium, thallium, ytterbium and lutetium, cobalt chloride, ferrous chloride, yttrium chloride, and mixtures thereof, organometallic compounds.
